(19) Europäisches Patentamt / European Patent Office / Office européen des brevets

(11) **EP 2 934 739 B1**

(12) **EUROPEAN PATENT SPECIFICATION**

(45) Date of publication and mention of the grant of the patent:
**02.10.2019 Bulletin 2019/40**

(51) Int Cl.:
***B01J 13/02*** *(2006.01)*

(21) Application number: **13818604.4**

(22) Date of filing: **17.12.2013**

(86) International application number:
**PCT/US2013/075714**

(87) International publication number:
**WO 2014/099946 (26.06.2014 Gazette 2014/26)**

(54) **ANHYDROUS POWDER-TO-LIQUID PARTICLES**

WASSERFREIE PULVER-ZU-FLÜSSIGKEITS-TEILCHEN

PARTICULES DE POUDRE-À-LIQUIDE ANHYDRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **19.12.2012 US 201213719649**

(43) Date of publication of application:
**28.10.2015 Bulletin 2015/44**

(73) Proprietor: **Johnson & Johnson Consumer Inc. Skillman, NJ 08558 (US)**

(72) Inventors:
• **FASSIH, Ali**
  **Franklin Park, NJ 08823 (US)**
• **SUN, Ying**
  **Belle Mead, NJ 08502 (US)**

(74) Representative: **Carpmaels & Ransford LLP One Southampton Row London WC1B 5HA (GB)**

(56) References cited:
**US-A1- 2010 266 647     US-A1- 2012 315 312**

## Description

The present disclosure relates to powder-to-liquid particles comprising a liquid core that is an emulsion substantially free of water, wherein the emulsion includes a continuous phase comprising a polar liquid that has a percent surface polarity of at least 24% and a dispersed phase comprising a hydrophobic component, surrounded by a shell comprising hydrophobic particles. The particles are stable in dry form and yet quickly transform into a liquid or cream-like form when subjected to shear. They can be advantageously formulated with other ingredients, particularly those unstable in the presence of water, into personal care compositions.

## BACKGROUND OF THE INVENTION

It is known that in the presence of a hydrophobic powder, such as a hydrophobic silicon dioxide powder (silicone-coated silica powder), water can be dispersed into fine droplets and enveloped by the hydrophobic material, thus preventing the droplets from rejoining. Such material has been described as "dry water," "powdered water," or "powder-to-liquid" and can have a water content of over 95%. It is formed by the intensive mixing of water with hydrophobic material. During this process water droplets are sheathed by the solid particles and prevented from flowing together again. The first experiments on the use of "dry water" as a cosmetic base date from the 1960's. See US 3,393,155. These free-flowing, fine powders liquefy when rubbed on the skin.

More recently, US 6,290,941 describes cosmetic or pharmaceutical powder-to-liquid compositions comprising hydrophobically coated silica particles into which are incorporated water and a water soluble polymer, the composition containing substantially no oil. Such compositions are said to require less silica while retaining the water-holding capacity, and permitting substantial elimination of added oil from the formula.

US 2012/0315312 A1 discloses core-shell particles with high content of glycerol.

WO 2011/075418 discloses a powdery composition comprising a) at least one powder in the form of core-shell particles, the core comprising liquid water or a liquid aqueous phase and the shell comprising hydrophobic or hydrophobized particles, and b) at least one powder comprising carrier, and b1) at least partially water soluble liquid and/or b2) a water reactive substrate each located in and/or on the carrier.

Eshtiaghi et al., Powder Technology, Vol.223, 2012, pages 65-76 describes a variety of powder-to-liquid materials and proposes mechanisms for their formation. Shell materials used included hydrophobic (silicone-coated) silica, hydrophobic glass beads and polytetrafluoroethylene (PTFE or TEFLON) powder. Core materials included water, glycerol, and polyethylene glycol (PEG). Reported particle sizes for materials containing glycerin were 1200 and 3400 microns.

Although water-based powder-to-liquids are commonly described, they are not suitable for formulating with active agents that are unstable or incompatible with water, e.g., plant extracts prone to oxidation and/or hydrolysis. In addition, water-containing particles generally lack structural stability and are prone to collapse or leak during storage, and allow evaporation of water from the core.

Applicants have now discovered a method of preparing powder-to-cream particles containing a core without water. Such particles are stable and useful for formulating with a variety of active agents, even those that are prone to oxidation and/or hydrolysis. Compositions containing such particles are also convenient to use while providing a cream-like, pleasant skin feel and skin substantivity (the ability to remain on the skin). The compositions can be used in cosmetic, skin care, wound care, dermatologic, and other personal care products, as well as in other applications and industries.

## SUMMARY OF THE INVENTION

The invention provides a powder comprising core/shell particles having an average particle size of less than 1000 microns, each core/shell particle comprising: 1) a liquid core that is an emulsion containing 5% by weight or less of water, wherein the emulsion includes a continuous phase comprising a polar liquid having a percent surface polarity of at least 24% as determined using the method of Fowkes referenced in the description and a dispersed phase comprising a hydrophobic component and 2) a shell comprising hydrophobic particles.

The invention also provides a personal care composition comprising the above powder.

## BRIEF DESCRIPTION OF THE FIGURES

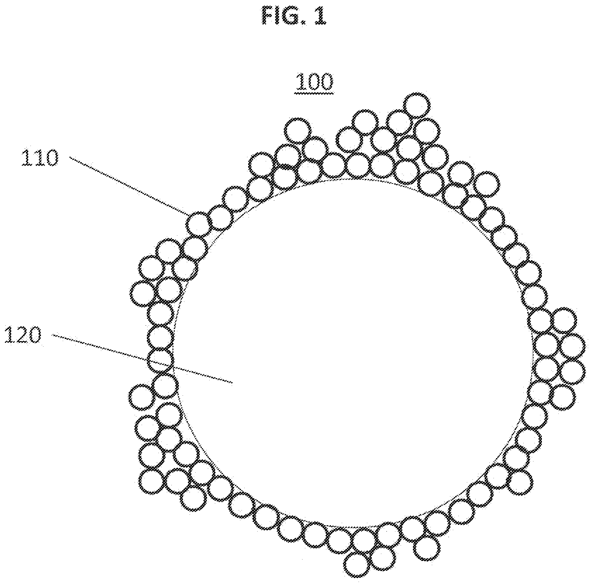

Comparative Figure 1 shows a schematic, cross-sectional view of a core/shell particle comprising a single phase liquid core.

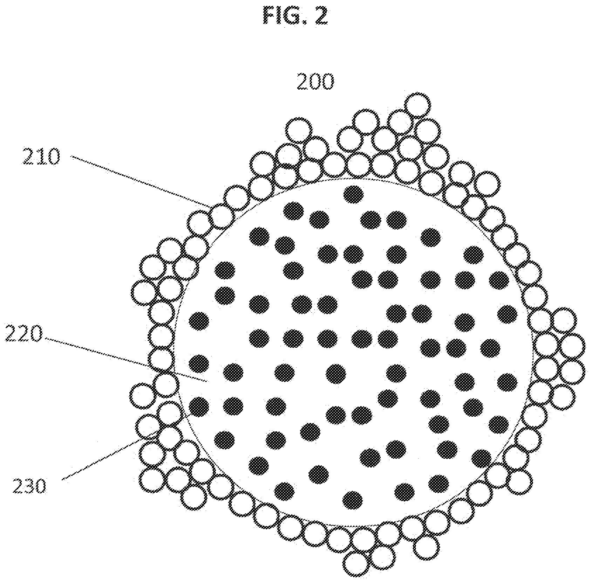

Figure 2 shows a schematic, cross-sectional view of a core/shell particle comprising a two-phase liquid core.

**DETAILED DESCRIPTION OF THE INVENTION**

As used herein, unless otherwise specified, all percentages are by weight based on the total weight of composition referred to.

As used herein, "substantially free" of an ingredient means containing 5% by weight or less of that ingredient. Preferably, substantially free of an ingredient means containing 2% or less, or 1% or less, or 0.5% or less or 0.1% or less, or 0.05% or less, or 0.01% or less, by weight of such ingredient. In certain embodiments, substantially free of an ingredient means completely free of the ingredient, *i.e.,* containing none of that ingredient.

As used herein, "active agent" is a compound (*e.g.,* a synthetic compound or a compound isolated from a natural source) that has a cosmetic or therapeutic effect on tissue (*e.g.,* a material capable of exerting a biological effect on the human body) such as therapeutic drugs or cosmetic agents. Examples of active agents include small molecules, peptides, proteins, nucleic acid materials, and nutrients such as minerals and extracts. The amount of the active agent used will depend on the active agent and/or the intended use of the end product. Active agents may be liquid, solid, or semi-solid. Further, active agents may be incorporated into the liquid core and/or the shell of the core/shell particles.

As used herein, "pharmaceutically acceptable," "cosmetically acceptable," or "dermatologically acceptable" means suitable for use in contact with tissues (*e.g.,* the skin, hair, mucosa, epithelium or the like) without undue toxicity, incompatibility, instability, irritation, or allergic response.

As used herein, "safe and effective amount" means an amount sufficient to provide a desired benefit at a desired level, but low enough to avoid serious side effects. The safe and effective amount of the ingredient or composition will vary with the area being treated, the age of the end user, the duration and nature of the treatment, the specific ingredient or composition employed, the particular carrier utilized, and like factors.

As used herein, the term "treating" or "treatment" means the alleviation or elimination of symptoms, cure, prevention, or inhibition of a disease or medical condition, or improvement of tissue growth/healing or cosmetic conditions such as reducing appearance of skin wrinkles/fine lines, under-eye bags, cellulites, skin marks/hyperpigmentation or uneven tone.

**Core/Shell Particles**

The powder of the invention comprises core/shell particles. Particles are defined by claim 1 and comprise a liquid core that is an emulsion substantially free of water and comprises a polar liquid. The polar liquid has a minimum surface polarity. The liquid core is surrounded by a shell of hydrophobic particles.

Comparative Figure 1 depicts a core/shell particle comprising a single phase liquid core. The particle **100** comprises a liquid core **120.** The liquid core is surrounded by a shell **110** of hydrophobic particles.

Figure 2 depicts a core/shell particle comprising a multi-phase (*e.g.,* two-phase) liquid core according to the invention. The particle **200** comprises an emulsion comprising a polar liquid **220** as the continuous (external) phase. The dispersed (internal) phase **230** comprises a hydrophobic material and/or solid particles. The liquid core is surrounded by a shell **210** of hydrophobic particles.

The hydrophobic particles of the shell are in the form of loose powder held together only by weak liquid-powder and powder-powder interactions via weak van der Waals forces. When subjected to slight forces on such as by rubbing with the hands, the core/shell particles collapse and the powder becomes a liquid, cream or gel.

Overall, the average particle size of the core/shell particles is less than 1000 micrometers, usually from 1 micrometer to 1000 micrometers, or 2 micrometers to 200 micrometers, or 3 micrometers to 100 micrometers, or 5 micrometers to 50 micrometers. The average particle size of the core/shell particles can be determined by any particle size measurement method for dry particles known to the art, such as optical microscopy, electron microscopy, or sieve analysis.

**The Core**

The liquid core is an emulsion containing 5% by weight or less of water, wherein the emulsion includes a continuous phase comprising a polar liquid having a percent surface polarity of at least 24% as determined using the method of Fowkes in the description.

As known in the art, the surface tension of a liquid (i.e., overall surface tension) is divided into two components, one representing a polar component and one representing a nonpolar (or dispersive) component. The polar component, "percent (%) surface polarity," is determined using the method of Fowkes described in Fowkes, Journal of Achievements in Materials and Manufacturing Engineering, 24, 1 (2007) 137-145.

Specifically, the overall surface tension of a sample is measured five times via the Wilhelmy plate method (described by Derelinch et. al. "Measurement of Interfacial Tension in Fluid-Fluid Systems", in Encyclopedia of Surface and Colloid Science, pages 3152-3166, Ed. By Arthur T. Hubbard, Marcel Dekker, Inc., 2002), using a Kruss Tensiometer K100. The plate used is a standard platinum plate of 19.9 mm x 0.2 mm perimeter.

The contact angle of each sample is also measured five times on a clean piece of poly(tertafluoroethylene) PTFE using a Kruss Drop Shape Analysis System DSA10. Measuring contact angle on PTFE is done as a means of separating the overall surface tension of each sample into polar and dispersive components. According to the Fowkes surface energy theory, the dispersive component of a liquid can be determined by knowing its overall surface tension and its contact angle against PTFE (which is a completely non-polar surface). The equation is as follows:

$$\sigma_L{}^D = \frac{\sigma_L{}^2 \left( \cos \theta_{PTFE} + 1 \right)^2}{72}$$

where $\theta_{PTFE}$ = the contact angle measured between PTFE and the sample liquid. The dispersive surface tension component ($\sigma_L{}^D$) can be determined for any liquid for which the overall surface tension ($\sigma_L$) is known simply by measuring the contact angle between that liquid and PTFE ($\theta_{PTFE}$) and using the equation above. The polar surface tension component for the liquid is then determined by difference ($\sigma_L{}^P = \sigma_L - \sigma_L{}^D$). The percent surface polarity is (%= $\sigma_L{}^P$ * 100%/ $\sigma_L$). See also F.M. Fowkes, Journal of Physical Chemistry, 67 (1963) 2538-2541.

The polar liquid has a percent surface polarity of at least 24%, or at least 25%, or at least 26%, or at least 30%.

The liquid core is substantially free of water, that is containing 5% by weight or less of water. The liquid core may be completely free of water, that is, anhydrous.

The liquid core is substantially free of preservatives. The liquid core may be completely free of preservatives.

The liquid core is an emulsion.

The liquid core is an emulsion comprising at least two phases. Such an emulsion comprises a continuous (external) phase comprising the polar liquid, and one or more dispersed (internal) phases comprising a hydrophobic component, such as an oil.

The liquid core is a multi-phase material, i.e. an emulsion. The polar liquid within such multi-phase material has a percent surface polarity of at least 24%, or at least 25%, or at least 26%, or at least 30%.

Emulsions for use as the liquid core may be prepared by conventional methods known in the art. Such methods typically involve mixing of the ingredients with a mixer or a homogenizer in one or more steps to achieve a stable and uniform multi-phase emulsion, with or without heating, cooling, application of vacuum, and the like.

The polar liquid may comprise one or more polyols. Such polyols include, but are not limited to glycerol (glycerin), polyglycerols, glycols, polyglycols, and mixtures thereof.

Examples of polyglycerols include, but are not limited to diglycerol (diglycerin), triglycerol (polyglcerin-3 or polyglycerol-3), tetraglycerol (polyglycerin-4 or polyglycerol-4), other polyglycerols (polycerol-n, where n > 4), and mixtures thereof.

Examples of glycols include, but are not limited to propylene glycol, ethylene glycol, butylene glycol and its isomers (e.g., 1,2-butanediol, 1,3-butanediol, 1,4-butanediol and 2,3-butanediol), hexylene glycol and its isomers, propanediol, dipropylene glycol, ethoxydiglycol, methylpropanediol, isopentyldiol, and mixtures thereof.

Examples of polyglycols include, but are not limited to, polyethylene glycol of various molecular weights, namely, molecular weights ranging from 300 g/mol to 10,000,000 g/mol, (*e.g.,* PEG-200, PEG-400, PEG-1000, PEG-2000 PEG-4000, PEG-6000), polypropylene glycol (PPG) of various molecular weights, and mixtures thereof.

The polar liquid may comprise a combination of a polyol with one or more other organic liquids. Such organic liquids include, but are not limited to alcohols, isosorbides, esters, ethers, lactones, and any organic compounds acceptable for therapeutic, cosmetic or personal product applications and capable of maintaining the percent surface polarity of the polar liquid at 24% or above.

Examples of alcohols include, but are not limited to ethyl alcohol, n-propyl alcohol, isopropyl alcohol, n-butyl alcohol, isobutyl alcohol, amyl alcohol, benzyl alcohol, octyldocanol, hexyldecanol, butyloctanol, and mixtures thereof.

Examples of isosorbides include, but are not limited to dimethyl isosorbide, diethyl isosorbide, ethylmethyl isosorbide, and mixtures thereof. Preferably, the isosorbide is an alkyl ester of isosorbide, such as dimethyl isosorbide.

Examples of esters include, but are not limited to benzyl benzoate, triacetin, glycerol trioctanoate, diethyl phthalate, and mixtures thereof.

Examples of ethers include, but are not limited to dicapryl ether, dipropylene glycol monomethyl ether, and mixtures thereof.

Examples of lactones include but are not limited to gluconolactone.

In one embodiment of the invention, the polar liquid comprises a mixture of a glycerol or polyglycerol with one or more glycols or polyglycols.

In an alternative embodiment of the invention, the polar liquid comprises a mixture of a glycerol or polyglycerol with one or more alcohols.

In yet another embodiment of the invention, the polar liquid comprises a mixture of a glycerol or polyglycerol with one or more isosorbides.

Preferably, the polar liquid comprises a glycerol, polyglycerol, or a mixture thereof. The amount of glycerol, polyglycerol, or mixture thereof may be 50% to 100%, or more than 70%, or more than 80%, or 85% or more, by weight of the polar liquid of the liquid core. The remainder of the liquid core may be one or more organic liquids such as non-glycerol polyols, alcohols, or isosorbides.

In one embodiment, the liquid core may further comprise at least one hydrophilic polymer, *e.g.,* natural or synthetic hydrophilic polymers. Such hydrophilic polymer may be soluble or partially soluble in the liquid core. Suitable hydrophilic polymers include, but are not limited to, homo-and copolymers of vinyl pyrrolidone (*e.g.,* PVP, or PVP/PVA copolymer), homo-or copolymers of vinyl alcohol (*e.g.,* polyvinyl alcohol or PVA), polyacrylamide, homo-or copolymers of acrylic and/or methacrylic acids, and salts and esters thereof (*e.g.,* CARBOPO/CARBOMER 934, 940, 941, 980, 1342, and 1382, and ULTREZ 10 and 21), cellulosic polymers (*e.g.,* hydroxymethylcellulose, hydroxyethyl cellulose, carboxy methyl cellulose, carboxy ethyl cellulose), polyurethanes, starch and its derivatives, and synthetic and natural gums (e.g., gum arabic or xanthan gum). Preferred hydrophilic polymers are acrylate polymers and copolymers, particularly polyacrylate neutralized by anhydrous neutralizers.

Incorporation of such polymers in the liquid core enhances interactions between the liquid core and the hydrophobic particles of the shell, thereby facilitating core-shell particle formation and improving the physical stability of the core/shell particles, which prevents premature particle collapse and liquid leakage during storage.

If used, the amount of the hydrophilic polymer is usually up to about 10%, or equal to or less than 5%, or equal to or less than 3%, or equal to or less than 2%, by weight of the liquid core.

In the emulsion, the liquid core may comprise any hydrophobic component as the dispersed phase. The hydrophobic component may be derived from animals, plants, or petroleum and may be natural or synthetic, for example as described in US 6,174,533.

The amount of hydrophobic component may be less than 80%, or equal to or less than 50%, or equal to or less than 30%, or equal to or less than 20%, by weight of the liquid core.

Nonlimiting examples of suitable hydrophobic components include:

(1) Mineral oil, which is also known as petrolatum liquid, is a mixture of liquid hydrocarbons obtained from petroleum.

(2) Petrolatum, which is also known as petroleum jelly, is a colloidal system of non-straight-chain solid hydrocarbons and high-boiling liquid hydrocarbons, in which most of the liquid hydrocarbons are held inside the micelles.

(3) Straight and branched chain hydrocarbons having from about 7 to about 40 carbon atoms. Nonlimiting examples of these hydrocarbon materials include dodecane, isododecane, squalane, cholesterol, hydrogenated polyisobutylene, docosane (*i.e,*. a C22 hydrocarbon), hexadecane, isohexadecane (such as commercially available hydrocarbon sold as PERMETHYL 101A by Presperse, South Plainfield, N.J.). Also useful are the C7-C40 isoparaffins, which are C7-C40 branched hydrocarbons.

(4) C1-C30 alcohol esters of C1-C30 carboxylic acids and of C2-C30 dicarboxylic acids, including straight and branched chain materials as well as aromatic derivatives (as used herein in reference to the hydrophobic component, mono-and poly-carboxylic acids include straight chain, branched chain and aryl carboxylic acids). Nonlimiting examples include diisopropyl sebacate, diisopropyl adipate, isopropyl myristate, isopropyl palmitate, methyl palmitate, myristyl propionate, 2-ethylhexyl palmitate, isodecyl neopentanoate, di-2-ethylhexyl maleate, cetyl palmitate, myristyl myristate, stearyl stearate, isopropyl stearate, methyl stearate, cetyl stearate, behenyl behenrate, dioctyl maleate, dioctyl sebacate, diisopropyl adipate, cetyl octanoate, diisopropyl dilinoleate.

(5) mono-, di-and tri-glycerides of C1-C30 carboxylic acids, *e.g.,* caprilic/capric triglyceride, PEG-6 caprylic/capric triglyceride, PEG-8 caprylic/capric triglyceride.

(6) alkylene glycol esters of C1-C30 carboxylic acids, *e.g.,* ethylene glycol mono-and di-esters, and propylene glycol mono-and di-esters of C1-C30 carboxylic acids *e.g.,* ethylene glycol distearate.

(7) propoxylated and ethoxylated derivatives of the foregoing materials.

(8) C1-C30 mono-and poly-esters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof.

(9) Organopolysiloxane oils. The organopolysiloxane oil may be volatile, non-volatile, or a mixture of volatile and non-volatile silicones. Nonlimiting examples of suitable organopolysiloxane oils include polyalkylsiloxanes, cyclic polyalkylsiloxanes, and polyalkylarylsiloxanes.

Polyalkylsiloxanes include polyalkylsiloxanes with viscosities of from about 0.5 to about 1,000,000 centistokes at 25° C. Commercially available polyalkylsiloxanes include the polydimethylsiloxanes, which are also known as dimethicones, examples of which include the VICASIL series sold by General Electric Company and the DOW CORNING 200 series sold by Dow Corning Corporation. Specific examples of suitable polydimethylsiloxanes include DOW CORNING 200 fluid, DOW CORNING 225, cetyl dimethicone, lauryl dimethicone, and cyclic polyalkylsiloxanes such as DOW CORNING 244, DOW CORNING 344, DOW CORNING 245, DOW CORNING 345 DOW CORNING 593 fluid, DOW CORNING 1401, 1402, and 1403 fluids.

(10) Vegetable oils and hydrogenated vegetable oils. Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated sesame oil, hydrogenated sunflower seed oil, and mixtures thereof.

(11) Animal fats and oils, *e.g.,* lanolin and derivatives thereof, cod liver oil.

(12) Also useful are C4-C20 alkyl ethers of polypropylene glycols, C1-C20 carboxylic acid esters of polypropylene glycols, and di-C8-C30 alkyl ethers. Nonlimiting examples of these materials include PPG-14 butyl ether, PPG-15 stearyl ether, dioctyl ether, dodecyl octyl ether, and mixtures thereof.

In general, the liquid core may contain any additional ingredients (*e.g.,* active agents or formulation excipients) soluble or dispersible in the polar liquid or its components, provided the additional ingredients do not impair the percent surface polarity of the liquid core. Pharmaceutically or cosmetically acceptable active agents or excipients, such as extracts of plants or minerals, natural or synthetic compounds of small molecular weight or polymers, acids or bases (particularly week acids or bases) for acidity adjustment, buffers, chelators, antioxidants, thickeners or gelling agents can be used.

In particular, active agents can be present in the liquid core.

For example in one embodiment, the liquid core comprises an emulsion in which an active agent is dissolved in the hydrophobic component of the emulsion. In another embodiment, an active agent that is hydrophobic may itself comprise the hydrophobic component of the emulsion.

Active agents such as methyl salicylate, essential oils, or organic sunscreens that are liquids at ambient temperature can be used in this manner.

The liquid core may comprise one or more emulsifying surfactants (emulsifiers) commonly used in pharmaceutical or cosmetic products.

In one embodiment, the liquid core comprises at least one polymeric surfactant having a molecular weight ranging from 1,000 Daltons to 50,000 Daltons, including, but not limited to, homo-polymers such as poly(ethylene oxide), poly(vinyl pyrrolidone) and poly(vinyl alcohol), block and graft copolymer polymeric surfactants such as diblock or triblock polymeric surfactants known as PLURONICS manufactured by BASF (Germany) or SYNPERONIC PE manufactured by ICI (U.K.) consisting of two poly-A blocks of poly(ethylene oxide)(PEO) and one block of poly(propylene oxide)(PPO), and diblocks of polystyrene-block-polyvinyl alcohol, triblocks of poly(methyl methacrylate)-block poly(ethylene oxide)-block poly(methyl methacrylate), diblocks of polystyrene block-polyethylene oxide and triblocks of polyethylene oxide-block polystyrene-polyethylene oxide, as well as amphipathic graft copolymer consisting of a polymeric backbone B (polystyrene or polymethyl methacrylate) and several A chains ("teeth") such as polyethylene oxide referred to as a "comb" stabilizer may be used.

The liquid core comprises at least one hydrophobically modified polysaccharide. Useful polysaccharides include sugars (*e.g.,* inulin), sugar analogs (*e.g.,* dextrans), starches (*e.g.,* starches from potato or tapioca), water-soluble celluloses (*e.g.,* hydroxypropylcellulose), hydrophobically modified inulin (polyfructose) as disclosed in US 6,534,647 (including commercially available INUTEC SP1 (ORAFTI, Tienen, Belgium), hydrophobically modified dextran as disclosed by O. Carrier et al. ("Inverse emulsions stabilized by a hydrophobically modified polysaccharide", Carbohydrate Polymers, 84(2011)599-604), hydrohobically modified starches from potato or tapioca as disclosed in US 8,258,250, US 7,417,020, US20110082105A1, and US 20110082290A1, (including commercially available NATURASURFTM PS-111, AKZO NOBEL CHEICALS INTERNATIONAL, B.V.), and hydrophobically modified water-soluble hydroxypropylcellulose as disclosed by C. Claro et al. ("Surface tension and rheology of aqueous dispersed systems containing a new hydrophobically modified polymer and surfactants", International Journal of Pharmaceutics, 347(2008)45-53). Other exemplary hydrophobically modified polysaccharides, include, but are not limited to, PEMULEN TR-1, PEMULEN TR-2, ETD 2020, CARBOPOL 1382 (Acrylates/C10-30 alkyl acrylate crosspolymer, by Noveon/Lubrizol, Cleveland, OH), NATROSOL CS Plus 330, 430, POLYSURF 67 (cetyl hydroxyethyl cellulose, Hercules, Wilmington, DE), ACULYN 22 (acrylates /steareth-20 methacrylate copolymer, Rohm & Haas, Philadelphia, PA), ACULYN 25 (acrylates/laureth-25 methacrylate Copolymer, Rohm & Haas), ACULYN 28 (acrylates /beheneth-25 methacrylate copolymer, Rohm & Haas), ACULYN 46 (PRG-150/stearyl alcohol/SMDI copolymer, Rohm & Haas), STABYLEN 30 (acrylates/vinyl isodecanoate, 3V-Sigma, Georgetown, SC), STRUCTURE 2001 (acrylates/steareth-20 itaconate copolymer, National Starch), STRUCTURE 3001 (acrylates/ceteth-20 itaconate copolymer, National Starch), STRUCTURE PLUS (acrylates/aminoacrylates/C10-30 alkyl PEG 20 itaconate copolymer, National Starch), QUATRISOFT LM-200 (polyquaternium-24, Amerchol,Greensburg, LA), CAPSULE, HI-CAP 100, N-CREAMER 46, CAPSUL TA, and N-LOK-1930 (all by Ingredion Incorporated, formally National Starch or Corn Products International, Inc.), Westchester, IL.

The amount of hydrophobically modified polysaccharide surfactant used is generally from 0.01% to 20%, or from 0.1% to 10%, or from 0.5% to 5%, or from 0.1% to 1%, by weight of the liquid core.

Other useful surfactants are described by T. Tadros ("Polymeric Surfactants in Disperse Systems", Advances in Colloid and Interface Science, 147-148,2009, page 281-299), and R.Y. Lochhead and S. Jones ("Polymers in Cosmetics: Recent Advances", Article 2004/07, Happi.com).

In one embodiment, the composition comprises at least one surfactant typically used to prepare oil-in-water (O/W) emulsions as disclosed in US 6,174,533.

The liquid core may comprise from 0.05% to 5%, or from 0.05% to 1%, by weight of such surfactant. Without intending to be limited by theory, it is believed that the surfactant assists in dispersing the hydrophobic component in the polar liquid. The surfactant, at a minimum, must be hydrophilic enough to disperse in the hydrophilic component. Preferred surfactants are those having an HLB of at least 8. The exact surfactant chosen will depend upon the pH of the composition and the other components present.

The surfactant can be any of the anionic surfactants, nonionic surfactants, amphoteric surfactants, zwitterionic surfactants cationic surfactants and mixtures clearly as are well known in the art.

Examples of nonionic surfactants that are useful herein are those that can be broadly defined as condensation products of long chain alcohols, e.g. C8-30 alcohols, with sugar or starch polymers, i.e., glycosides. These compounds can be represented by the formula $(S)_n$-O-R wherein S is a sugar moiety such as glucose, fructose, mannose, and galactose; n is an integer of from about 1 to about 1000, and R is a C8-30 alkyl group. Examples of long chain alcohols from which the alkyl group can be derived include decyl alcohol, cetyl alcohol, stearyl alcohol, lauryl alcohol, myristyl alcohol, oleyl alcohol, and the like. Examples of these surfactants include those wherein S is a glucose moiety, R is a C8-20 alkyl group, and n is an integer of from about 1 to about 9. Commercially available examples of these surfactants include decyl polyglucoside (available as APG 325 CS from Henkel) and lauryl polyglucoside (available as APG 600 CS and 625 CS from Henkel).

Other useful nonionic surfactants include the condensation products of alkylene oxides with fatty acids (i.e. alkylene oxide esters of fatty acids). These materials have the general formula $RCO(X)_nOH$ wherein R is a C10-30 alkyl group, X is $-OCH_2CH_2-$ (i.e. derived from ethylene glycol or oxide) or $-OCH_2CHCH_3-$ (i.e. derived from propylene glycol or oxide), and n is an integer from about 6 to about 200. Other nonionic surfactants are the condensation products of alkylene oxides with 2 moles of fatty acids (i.e. alkylene oxide diesters of fatty acids). These materials have the general formula $RCO(X)_nOOCR$ wherein R is a C10-30 alkyl group, X is $-OCH_2CH_2-$ (i.e., derived from ethylene glycol or oxide) or $-OCH_2CHCH_3-$ (i.e. derived from propylene glycol or oxide), and n is an integer from about 6 to about 100. Other nonionic surfactants are the condensation products of alkylene oxides with fatty alcohols (i.e. alkylene oxide ethers of fatty alcohols). These materials have the general formula $R(X)_nOR'$ wherein R is a C10-30 alkyl group, X is $-OCH_2CH_2-$ (i.e. derived from ethylene glycol or oxide) or $-OCH_2CHCH_3-$ (i.e. derived from propylene glycol or oxide), and n is an integer from about 6 to about 100 and R' is H or a C10-30 alkyl group. Still other nonionic surfactants are the condensation products of alkylene oxides with both fatty acids and fatty alcohols (i.e. wherein the polyalkylene oxide portion is esterified on one end with a fatty acid and etherified (i.e. connected via an ether linkage) on the other end with a fatty alcohol). These materials have the general formula $RCO(X)_nOR'$ wherein R and R' are C10-30 alkyl groups, X is $-OCH_2CH_2$ (i.e., derived from ethylene glycol or oxide) or $-OCH_2CHCH_3$ --(derived from propylene glycol or oxide), and n is an integer from about 6 to about 100. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Other nonionic surfactants suitable for use herein include sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-C30 ethers of polyols, alkyl phosphates, polyoxyalkylene fatty ether phosphates, fatty acid amides, acyl lactylates, and mixtures thereof. Nonlimiting examples of these non-silicon-containing emulsifiers include: polyethylene glycol 20 sorbitan monolaurate (Polysorbate 20 or TWEEN 20), polyethylene glycol 5 soya sterol, Steareth-20, Ceteareth-20, PPG-2 methyl glucose ether distearate, Ceteth-10, Polysorbate 80 (TWEEN 80), Polysorbate 40 (TWEEN 40), cetyl phosphate, potassium cetyl phosphate, diethanolamine cetyl phosphate, Polysorbate 60 (TWEEN 60), glyceryl stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

Other emulsifiers useful herein are fatty acid ester blends based on a mixture of sorbitan or sorbitol fatty acid ester and sucrose fatty acid ester, the fatty acid in each instance being preferably C8-C24, more preferably C10-C20. The preferred fatty acid ester emulsifier is a blend of sorbitan or sorbitol C 16-C 20 fatty acid ester with sucrose C10-C16 fatty acid ester, especially sorbitan stearate and sucrose cocoate. This is commercially available from ICI under the trade name ARLATONE 2121.

The surfactants useful herein can alternatively or additionally include any of a wide variety of cationic, anionic, zwitterionic, and amphoteric surfactants such as are known in the art. Cationic surfactants useful herein include cationic ammonium salts such as quaternary ammonium salts, and amino-amides. Nonlimiting examples of anionic surfactants include the alkoyl isethionates (*e.g.,* C12-C30), alkyl and alkyl ether sulfates and salts thereof, alkyl and alkyl ether phosphates and salts thereof, alkyl methyl taurates (*e.g.,* C12-C30), and soaps (*e.g.,* alkali metal salts, *e.g.,* sodium or potassium salts) of fatty acids.

Amphoteric and zwitterionic surfactants are also useful herein. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C8-C18) and one contains an anionic water solubilizing group, *e.g.,* carboxy, sulfonate, sulfate, phosphate, or phosphonate. Examples are alkyl imino acetates, and iminodialkanoates and aminoalkanoates, imidazolinium and ammonium derivatives. Other suitable amphoteric and zwitterionic surfactants are those selected from the group consisting of betaines, sultaines, hydroxysultaines, alkyl sarcosinates (*e.g.,* C12-C30), and alkanoyl sarcosinates.

The emulsions of the present invention may include a silicone containing emulsifier or surfactant. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds which contain C2-C30 pendant side chains. Still other useful dimethicone copolyols include materials having various cationic, anionic, amphoteric, and zwitterionic pendant moieties.

Dimethicone copolyol emulsifiers may be also be used. Nonlimiting examples of dimethicone copolyols and other silicone surfactants useful as emulsifiers herein include polydimethylsiloxane polyether copolymers with pendant polyethylene oxide sidechains, polydimethylsiloxane polyether copolymers with pendant polypropylene oxide sidechains, polydimethylsiloxane polyether copolymers with pendant mixed polyethylene oxide and polypropylene oxide sidechains, polydimethylsiloxane polyether copolymers with pendant mixed poly(ethylene)(propylene)oxide sidechains, polydimethylsiloxane polyether copolymers with pendant organobetaine sidechains, polydimethylsiloxane polyether copolymers with pendant carboxylate sidechains, polydimethylsiloxane polyether copolymers with pendant quaternary ammonium sidechains; and also further modifications of the preceding copolymers containing pendant C2-C30 straight, branched, or cyclic alkyl moieties. Examples of commercially available dimethicone copolyols useful herein sold by Dow Corning Corporation are DOW CORNING 190, 193, Q2-5220, 2501 Wax, 2-5324 fluid, and 3225C (the later material being sold as a mixture with cyclomethicone). Cetyl dimethicone copolyol is commercially available as a mixture with polyglyceryl-4 isostearate (and) hexyl laurate and is sold under the tradename ABIL® WE-09 (available from Goldschmidt). Cetyl dimethicone copolyol is also commercially available as a mixture with hexyl laurate (and) polyglyceryl-3 oleate (and) cetyl dimethicone and is sold under the tradename ABIL® WS-08 (also available from Goldschmidt). Other nonlimiting examples of dimethicone copolyols also include lauryl dimethicone copolyol, dimethicone copolyol acetate, dimethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate.

**The Shell**

The shell comprises hydrophobic particles. As used herein, "hydrophobic" includes both hydrophobic particles *per se* and hydrophobized particles obtained by reaction of the surface of hydrophilic particles with a hydrophobic surface modifying agent.

Useful hydrophobized particles include, but are not limited to, silicone- or silane-coated powders, or fluoropolymer-coated powders, such as talc, kaolin, mica, sericite, dolomite, phlogopite, synthetic mica, lepidolite, biotite, lithia mica, vermiculite, magnesium carbonate, calcium carbonate, aluminum silicate, barium silicate, calcium silicate, magnesium silicate, strontium silicate, tungstenic acid metal salts, magnesium, silica, zeolite, barium sulfate, calcined calcium sulfate, calcium phosphate, fluorapatite, hydroxyapatite, titania, fumed titania, zinc oxide, alumina and fumed alumina. Other hydrophobic particles include, but are not limited to, particles of hydrophobic compounds or polymers, such as solid long chain fatty acids and their esters, alcohols, and metal salts (*e.g.,* stearic acid, stearyl alcohol, and magnesium stearate), hydrophobic waxes (*e.g.,* paraffin wax and beeswax), and fluoropolymers (*e.g.,* polyvinylfluoride, polyvinylidene fluoride, polytetrafluoroethylene, polychlorotrifluoroethylene, perfluoroalkoxy polymer, fluorinated ethylenepropylene, polyethylenetetrafluoroethylene, polyethylenechlorotrifluoroethylene, perfluorinated elastomer, fluorocarbon, chlorotrifluoroethylenevinylidene fluoride and, perfluoropolyether.

Among these, hydrophobized silica particles that form a three dimensional network, an aggregated structure, are a preferred shell material. The silica may be a precipitated silica or a fumed silica, the latter being preferred. Fumed silica is obtained in a flame hydrolysis or flame oxidation process. Its purity is higher than 99 wt %, usually higher than 99.8 wt %. Fumed silica usually forms a three-dimensional network of aggregated primary particles and are porous. The fumed silica primary particles bear hydroxyl groups at their surface and are nonporous.

Other hydrophobic fumed metal oxides may also be used, such as hydrophobic fumed titanium oxide and aluminum oxide, such as Aeroxide. $TiO_2$ T805, and Aeroxide Alu C805 (both from EVONIK, Piscataway, NJ).

Precipitated and fumed silica particles, as well as other hydrophilic particles may be hydrophobized in a subsequent step. Procedures for this step are known for the person skilled in the art.

WO2011/076518 discloses these and other hydrophobic or hydrophobized silica particles suitable for use as the shell material of the present invention.

Hydrophobic surface modifying agents include silanes, including organosilanes, holoorganosilanes, and cyclinc polysiloxanes, which may be used individually or as a mixture. Examples of hydrophobic surface modifying agents include octyltrimethoxysilane, octyltriethoxysilane, hexamethyldisilazane, hexadecyltrimethoxysilane, hexadecyltriethoxysilane, dimethylpolysiloxane, nonafluorohexyltrimethoxysilane, tridecafluorooctyltrimethoxysilane, tridecafluorooctyltriethoxysilane. With particular preference it is possible to use hexamethyldisilazane, octyltriethoxysilane and dimethyl polysiloxanes.

The hydrophobic particles may be hydrophobized silica particles having a BET surface area of 30 $m^2$/g to 500 $m^2$/g, or 100 $m^2$/g to 350 $m^2$/g. Due to the reaction with the surface modifying agent these particles may contain 0.1 to 15 wt %, usually 0.5 to 5 wt %, of carbon.

Examples of useful hydrophobic particles include AEROSIL® R104 (octamethylcyclotetrasiloxane; 150 m2/g; 55); AEROSIL® R106 (octamethylcyclotetrasiloxane; 250 m2/g; 50), AEROSIL® R202 (polydimethylsiloxane; 100 $m_2$/g; 75), AEROSIL ® R805 (octylsilane; 150 m2/g; 60), AEROSIL® R812 (hexamethyldisilazane; 260 $m^2$/g; 60), AEROSIL® R812S (hexamethyldisilazane; 220 $m^2$/g; 65), AEROSIL® R8200 (hexamethyldisilazane; 150 $m^2$/g; 65). The indications in parenthesis refer to the surface modifying agent, the approximate BET surface area and the approximate methanol wettability.

It may also be beneficial to use hydrophobized fumed silica particles in compacted form or as granules.

Other suitable hydrophobic particles include fine inorganic, organic, or polymeric fine powders coated with silicone, silane or fluoro-compounds, which can be used alone or as mixture with hydrophobic silica or hydrophobic fumed silica powder.

The amount of hydrophobic particles in the powder is 2% to 30%, or 2.5% to 20%, or 3% to 10%, or 3% to 8%, by weight based on the total weight of powder (comprising core/shell particles).

In one embodiment, the shell consists of hydrophobized fumed silica particles that are obtained by reacting a hydrophilic fumed silica having a BET surface area of from 30 to 500 $m^2$/g.

In another embodiment, the hydrophobized fumed silica particles are obtained by reacting a hydrophilic fumed silica having a BET surface area from 270 to 330 $m^2$/g with hexamethyldisilazane to give hydrophobized fumed silica particles having a BET surface area of from 200 to 290 $m^2$/g and a carbon content of 2 to 4 wt % and methanol wettability of at least 50.

In one embodiment, active agents and/or additional ingredients are present in the shell.

**Second Powder**

The powder comprising core/shell particles may be mixed with a second powder. The mixing process is usually done during the product manufacturing process. However, the mixing process may also be carried out post-manufacturing by a user prior to use. In this case, the second powder and powder comprising core/shell particles may be packaged in a dual chamber container or separate containers.

In one embodiment, the second powder comprises one or more solid active agents, liquid actives impregnated into absorbent powder materials, solid cosmetic/pharmaceutical formulation excipients, or liquid cosmetic/pharmaceutical formulation excipients impregnated into absorbent powder materials.

Solid active agents that may be used in the second powder include unstable actives such as certain vitamins (e.g., ascorbic acid), and natural extracts containing antioxidants suitable for use in the compositions of this invention, include, but not limited to plant extracts containing flavonoids, phenolic compounds, flavones, flavanones, isoflavonoids, mono, di- and triterpenes, sterols and their derivatives. Examples of such plant extracts include grape seed, green tea, pine bark and propolis extracts and legume extracts and the like..

Absorbent powder materials include pharmaceutically or cosmetically acceptable porous powders such as silica and fumed silica, powders of starches, and clays, synthetic and natural fibers, as well as the materials described as useful for the hydrophobic particles of the shell.

**Method of Making Core/Shell Particles**

A single phase liquid core can be made by simple blending or mixing of the liquid ingredients until uniform. High shearing is not required for this step, as blending of miscible liquids does not require high energy. The liquid mixing for this step may be done with equipment such as blenders, lab scale mixers, or homogenizers. The sample may be heated in cases where an active agent contained therein requires higher temperature to dissolve in the liquid mixture. The resulting homogeneous liquid can be converted to a powder by mixing with the hydrophobic particles of the shell under high shear, such as with a blender or a rotor-stator mixer or other inline high rotational speed mixers. It is preferred to run the powderization step with all contents at room temperature or below.

In the case of a liquid core comprising an emulsion, the ingredients of the liquid core, including immiscible liquids, and/or active agents, and emulsifiers are mixed together under high shear until an emulsion is formed. The mixing for this step may be done with equipment such as blenders or homogenizers. The sample may be heated in cases where the active agent requires a higher temperature to dissolve in the liquid. The resulting emulsion can be converted to a powder by mixing with the hydrophobic particles under high shear, such as with a blender or a rotor-stator mixer or other inline high rotational speed mixers. It is preferred to run the powderization step with all contents at room temperature or below.

Methods of using high rotational speed mixers for powder-liquid mixing to prepare the core/shell compositions are known in the art. The energy of mixing should be high enough to break the liquid into fine droplets to be covered or encapsulated by the hydrophobic powder shell. L. Forny et. al. ("Influence of mixing characteristics for water encapsulation by self-assembling hydrophobic silica nanoparticles," Powder Technology 189, 2009, pages 263-269) describe the method and requirements for such preparation

**Use**

The powder comprising core/shell particles has great versatility in application, and can be used in many consumer and medical products for human and animal use such as ingestible compositions (such as tablets and capsules), topical compositions (such as creams, lotions, gels, shampoos, cleansers, powders patches, bandages, and masks for application to the skin or mucosal membranes), garments (such as undergarments, underwear, bras, shirts, pants, pantyhose, socks, head caps, facial masks, gloves, and mittens), linens (such as towels, pillow covers or cases and bed sheets), sanitizing products for household and clinical settings, microcides for plants, and devices (such as toothbrushes, dental flosses, periodontal implants or inserts, orthodontic braces, joint wraps/supports, buccal patches, ocular inserts or implants such as contact lenses, nasal implants or inserts, and contact lens cleaning products, wound dressings, diapers, sanitary napkins, wipes, tampons, rectal and vaginal suppositories, and in coatings or embedded surfaces on medical devices and other surfaces where antimicrobial or other beneficial effects are desired).

The powder comprising core/shell particles can be incorporated onto fibers, nonwovens, hydrocolloids, adhesives, films, polymers, and other substrates. In one embodiment, the powder is in contact with a tissue interface. Methods of applying the powder on substrates include electrostatic spray coating, mechanical sieving, co-extrusion, adhesive spraying.

The powder comprising core/shell particles may contain a wide range of active agents used for various applications as described in the sections below.

The powder comprising core/shell particles may be administered topically, locally (via buccal, nasal, retal or vaginal route), or systemically (*e.g.,* peroral route) to a subject (*e.g.,* a human) in need of treatment for a condition or disease, or to otherwise provide a therapeutic effect. Such therapeutic effects include, but are not limited to: antimicrobial effects (*e.g.,* antibacterial, antifungal, antiviral, and anti-parasitic effects); anti-inflammation effects including effects in the superficial or deep tissues (*e.g.,* reduce or elimination of soft tissue edema or redness); elimination or reduction of pain, itch or other sensory discomfort; regeneration or healing enhancement of hard tissues (*e.g.,* enhancing growth rate of the nail or regrowth of hair loss due to alopecia) or increase soft tissue volume (*e.g.,* increasing collagen or elastin in the skin or lips); increasing adepocyte metabolism or improving body appearance (*e.g.,* effects on body contour or shape, and cellulite reduction); and increasing circulation of blood or lymphocytes.

The powder comprising core/shell particles may be combined with one or more other active agents not contained in a second powder.

In one embodiment, a composition containing the powder comprising core/shell particles further contains a safe and effective amount of an active agent, for example, from 0.001 percent to 20 percent, such as from 0.01 percent to 10 percent, by weight of the composition of the active agent.

**Topical Skin Compositions**

In one embodiment, the invention provides a topical composition containing the powder comprising core/shell particles that is suitable for administering to mammalian skin, such as human skin. In one embodiment, such topical composition contains a safe and effective amount of (i) the powder comprising core/shell particles, and (ii) a cosmetically- or pharmaceutically-acceptable carrier.

The topical compositions may be made into a wide variety of products that include but are not limited to leave-on products (such as lotions, creams, gels, sticks, sprays, and ointments), skin cleansing products (such as liquid washes, solid bars, and wipes), hair products (such as shampoos, conditioners, sprays, and mousses), shaving creams, film-forming products (such as masks), make-up (such as foundations, eye liners, and eye shadows), deodorant and anti-perspirant compositions, and the like. These product types may contain any of several cosmetically- or pharmaceutically-acceptable carrier forms including, but not limited to solutions, suspensions, emulsions such as microemulsions and nanoemulsions, gels, and solids carrier forms. Other product forms can be formulated by those of ordinary skill in the art.

In one embodiment, the topical composition is used for the treatment of skin conditions. Examples of such skin conditions include, but are not limited to acne (*e.g.,* blackheads and whiteheads), rosacea, nodule-cystic, and other microbial infections of the skin; visible signs of skin aging (*e.g.,* wrinkles, sagging, sallowness, and age-spots); loose or lax skin, folliculitis and pseudo-folliculitis barbae; excess sebum (*e.g.,* for sebum reduction or oily/shining skin appearance inhibition or control); pigmentation (*e.g.,* for reduction of hyperpigmentation such as freckles, melasma, actinic and senile lentigines, age-spots, post-inflammatory hypermelanosis, Becker's naevus, and facial melanosis or enhancing the pigmentation of light skin); excess hair growth (*e.g.,* skin on the leg), or insufficient hair growth (*e.g.,* on the scalp); dermatitis (*e.g.,* atopic, contact, or seborrheic dermatitis), dark circles under the eye, stretch marks, cellulite, excessive sweating (*e.g.,* hyperhidrosis), and/or psoriasis.

(a) Topical Anti-Acne/Anti-Rosacea Compositions

In one embodiment, the topical composition also contains an anti-acne and/or anti-rosacea active agent. Examples of anti-acne and anti-rosacea agents include, but are not limited to: retinoids such as tretinoin, isotretinoin, motretinide, adapalene, tazarotene, azelaic acid, and retinol; salicylic acid; benzoyl peroxide; resorcinol; sulfur; sulfacetamide; urea; antibiotics such as tetracycline, clindamycin, metronidazole, and erythromycin; anti-inflammatory agents such as corticosteroids (*e.g.,* hydrocortisone), ibuprofen, naproxen, and hetprofen; and imidazoles such as ketoconazole and elubiol; and salts and prodrugs thereof. Other examples of anti-acne active agents include essential oils, alpha-bisabolol, dipotassium glycyrrhizinate, camphor, β-glucan, allantoin, feverfew, flavonoids such as soy isoflavones, saw palmetto, chelating agents such as EDTA, lipase inhibitors such as silver and copper ions, hydrolyzed vegetable proteins, inorganic ions of chloride, iodide, fluoride, and their nonionic derivatives chlorine, iodine, fluorine, and synthetic phospholipids and natural phospholipids such as ARLASILK™ phospholipids CDM, SV, EFA, PLN, and GLA (commercially available from Uniqema, ICI Group of Companies, Wilton, UK).

(b) Topical Anti-Aging Compositions

In one embodiment, the topical composition also contains an anti-aging agent. Examples of suitable anti-aging agents include, but are not limited to inorganic sunscreens such as titanium dioxide and zinc oxide; organic sunscreens such as octyl-methoxy cinnamates; retinoids; dimethylaminoethanol (DMAE), copper containing peptides, vitamins such as vitamin E, vitamin A (retinol and its derivatives, e.g., retinyl palmitate), vitamin C (ascorbic acid and its derivative, e.g., Ascorbic Acid 2-Glucoside/AA2G), and vitamin B (e.g., niacinamide, niacin) and vitamin salts or derivatives such as ascorbic acid di-glucoside and vitamin E acetate or palmitate; alpha hydroxy acids and their precursors such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucoheptonic acid, glucoheptono 1,4-lactone, gluconic acid, gluconolactone, glucuronic acid, glucuronolactone, isopropyl pyruvate, methyl pyruvate, mucic acid, pyruvic acid, saccharic acid, saccharic acid 1,4-lactone, tartaric acid, and tartronic acid; beta hydroxy acids such as beta-hydroxybutyric acid, beta-phenyl-lactic acid, and beta-phenylpyruvic acid; tetrahydroxypropyl ethylene-diamine, N,N,N',N'-Tetrakis(2-hydroxypropyl)ethylenediamine (THPED); and botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, and safflower; and salts and prodrugs thereof.

(c) Topical Depigmentation Compositions

In one embodiment, the topical composition contains a depigmentation agent. Examples of suitable depigmentation agents include, but are not limited to: soy extract; soy isoflavones; retinoids such as retinol; kojic acid; kojic dipalmitate; hydroquinone; arbutin; transexamic acid; vitamins such as niacinamide, niacin and vitamin C (ascorbic acid and AA2G; azelaic acid; linolenic acid and linoleic acid; placertia; licorice; and extracts such as chamomile, grape seeds and green tea; and salts and prodrugs thereof.

(d) Topical Antipsoriatic Compositions

In one embodiment, the topical composition contains an antipsoriatic active agent. Examples of antipsoriatic active agents (e.g., for seborrheic dermatitis treatment) include, but are not limited to, corticosteroids (e.g., betamethasone dipropionate, betamethasone valerate, clobetasol propionate, diflorasone diacetate, halobetasol propionate, triamcinonide, dexamethasone, fluocinonide, fluocinolone acetonide, halcinonide, triamcinolone acetate, hydrocortisone, hydrocortisone verlerate, hydrocortisone butyrate, aclometasone dipropionte, flurandrenolide, mometasone furoate, methylprednisolone acetate), methotrexate, cyclosporine, calcipotriene, anthraline, shale oil and derivatives thereof, elubiol, ketoconazole, coal tar, salicylic acid, zinc pyrithione, selenium sulfide, hydrocortisone, sulfur, menthol, and pramoxine hydrochloride, and salts and prodrugs thereof

(e) Other Topical Ingredients

In one embodiment, the topical composition contains a plant extract as an active agent. Examples of plant extracts include, but are not limited to, feverfew, soy, glycine soja, oatmeal, what, aloe vera, cranberry, witch-hazel, alnus, arnica, artemisia capillaris, asiasarum root, birch, calendula, chamomile, cnidium, comfrey, fennel, galla rhois, hawthorn, houttuynia, hypericum, jujube, kiwi, licorice, magnolia, olive, peppermint, philodendron, salvia, sasa albomarginata, natural isoflavonoids, soy isoflavones, and natural essential oils.
In one embodiment, the topical composition contains one or more buffering agents such as citrate buffer, phosphate buffer, lactate buffer, gluconate buffer, or gelling agent, thickener, or polymer.
In one embodiment, the composition or product contains a fragrance effective for reducing stress, calming, and/or affecting sleep such as lavender and chamomile.

**Topical Mucosal Compositions**

In one embodiment, the topical composition is suitable for administering to a mucosal membrane, such as human oral, rectal, or vaginal mucosal membrane. Such composition contains a safe and effective amount of (i) the powder comprising core/shell particles and (ii) a cosmetically- or pharmaceutically-acceptable carrier.
Such compositions may be made into a wide variety of products for application to mucosa, including but not limited to vaginal creams, tampons, suppositories, floss, mouthwash, toothpaste. Other product forms can be formulated by those of ordinary skill in the art.
In one embodiment, the composition is used for the treatment of a mucosal membrane condition. Examples of such treatments include, but are not limited to, treatment of vaginal candidiasis and vaginosis, genital and oral herpes, cold sore, canker sore, oral hygiene, periodontal disease, teeth whitening, halitosis, prevention of biofilm attachment, and other microbial infections of the mucosa.
The powder comprising core/shell particles can be incorporated into compositions for the treatment of candidiasis with actives such as, but not limited to tioconazole; clotrimazole; and nystatin.
The powder comprising core/shell particles can be incorporated into compositions for the treatment of bacterial vaginosis with actives such as, but not limited to, clindamycin hydrochloride and metronidazole.

The powder comprising core/shell particles can be incorporated into compositions for the treatment of periodontal disease with actives such as, but not limited to minocycline.

**Topical Compositions for Treatment of Wounds, Lesions and Scars**

In one embodiment, the powder comprising core/shell particles is incorporated into wound dressings or bandages to provide healing enhancement or scar prevention. Wounds or lesions that may be treated include, but are not limited to acute wounds as well as chronic wounds including diabetic ulcer, venus ulcer, and pressure sores.

In one embodiment, the wound dressing or bandage contains an active agent commonly used as for topical wound and scar treatment, such as antibiotics, anti-microbials, wound healing enhancing agents, antifungal drugs, anti-psoriatic drugs, and anti-inflammatory agents.

Examples of antifungal drugs include but are not limited to miconazole, econazole, ketoconazole, sertaconazole, itraconazole, fluconazole, voriconazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, and their pharmaceutically acceptable salts and prodrugs. In one embodiment, the antifungal drug is an azole, an allylamine, or a mixture thereof.

Examples of antibiotics (or antiseptics) include but are not limited to mupirocin, neomycin sulfate bacitracin, polymyxin B, 1-ofloxacin, tetracyclines (chlortetracycline hydrochloride, oxytetracycline-10 hydrochloride and tetrachcycline hydrochloride), clindamycin phosphate, gentamicin sulfate, metronidazole, hexylresorcinol, methylbenzethonium chloride, phenol, quaternary ammonium compounds, tea tree oil, and their pharmaceutically acceptable salts and prodrugs.

Examples of antimicrobials include but are not limited to salts of chlorhexidine, such as lodopropynyl butylcarbamate, diazolidinyl urea, chlorhexidene digluconate, chlorhexidene acetate, chlorhexidene isethionate, and chlorhexidene hydrochloride. Other cationic antimicrobials may also be used, such as benzalkonium chloride, benzethonium chloride, triclocarbon, polyhexamethylene biguanide, cetylpyridium chloride, methyl and benzethonium chloride. Other antimicrobials include, but are not limited to: halogenated phenolic compounds, such as 2,4,4',-trichloro-2-hydroxy diphenyl ether (Triclosan); parachlorometa xylenol (PCMX); and short chain alcohols, such as ethanol, propanol, and the like. In one embodiment, the alcohol is at a low concentration (*e.g.,* less than about 10% by weight of the carrier, such as less than 5% by weight of the carrier) so that it does not cause undue drying of the barrier membrane.

Examples of anti-viral agents for viral infections such as herpes and hepatitis, include, but are not limited to, imiquimod and its derivatives, podofilox, podophyllin, interferon alpha, acyclovir, famcyclovir, valcyclovir, reticulos and cidofovir, and salts and prodrugs thereof.

Examples of anti-inflammatory agents include, but are not limited to, suitable steroidal anti-inflammatory agents such as corticosteroids such as hydrocortisone, hydroxyltriamcinolone alphamethyl dexamethasone, dexamethasonephosphate, beclomethasone dipropionate, clobetasol valerate, desonide, desoxymethasone, desoxycorticosterone acetate, dexamethasone, dichlorisone, diflorasone diacetate, diflucortolone valerate, fluadrenolone, fluclarolone acetonide, fludrocortisone, flumethasone pivalate, fluosinolone acetonide, fluocinonide, flucortine butylester, fluocortolone, fluprednidene (fluprednylidene)acetate, flurandrenolone, halcinonide, hydrocortisone acetate, hydrocortisone butyrate, methylprednisolone, triamcinolone acetonide, cortisone, cortodoxone, flucetonide, fludrocortisone, difluorosone diacetate, fluradrenalone acetonide, medrysone, amciafel, amcinafide, betamethasone, chlorprednisone, chlorprednisone acetate, clocortelone, clescinolone, dichlorisone, difluprednate, flucloronide, flunisolide, fluoromethalone, fluperolone, fluprednisolone, hydrocortisone valerate, hydrocortisone cyclopentylproprionate, hydrocortamate, meprednisone, paramethasone, prednisolone, prednisone, beclomethasone dipropionate, betamethasone dipropionate, triamcinolone, and salts are prodrugs thereof. In one embodiment, the steroidal anti-inflammatory for use in the present invention is hydrocortisone. A second class of anti-inflammatory agents which is useful in the compositions of the present invention includes the nonsteroidal anti-inflammatory agents.

Examples of wound healing enhancing agents include recombinant human platelet-derived growth factor (PDGF) and other growth factors, ketanserin, iloprost, prostaglandin E1 and hyaluronic acid, scar reducing agents such as mannose-6-phosphate, analgesic agents, anesthetics, hair growth enhancing agents such as minoxadil, hair growth retarding agents such as eflornithine hydrochloride, antihypertensives, drugs to treat coronary artery diseases, anticancer agents, endocrine and metabolic medication, neurologic medications, medication for cessation of chemical additions, motion sickness, protein and peptide drugs.

**Topical Treatment of Microbial Infections of the Body**

In one embodiment, the powder comprising core/shell particles is used, with or without other antifungal active agents, to treat or prevent fungal infections (*e.g.,* dermatophytes such as trichophyton mentagrophytes), including, but not limited to, onychomycosis, sporotrichosis, tinea unguium, tinea pedis (athlete's foot), tinea cruris (jock itch), tinea corporis (ringworm), tinea capitis, tinea versicolor, and candida yeast infection-related diseases (*e.g.,* candida albicans) such as diaper rash, oral thrushm, cutaneous and vaginal candidiasis, genital rashes, Malassezia furfur infection-related diseases such as Pityriasis versicolor, Pityriasis folliculitis, seborrhoeic dermatitis, and dandruff.

In another embodiment, the powder comprising core/shell particles is used, with or without other antibacterial active agents, to treat and prevent bacterial infections, including, but not limited to, acne, cellulitis, erysipelas, impetigo, folliculitis, and furuncles and carbuncles, as well as acute wounds and chronic wounds (venous ulcers, diabetic ulcers and pressure ulcers).

In another embodiment, the powder comprising core/shell particles is used, with or without other antiviral active agents, to treat and prevent viral infections of the skin and mucosa, including, but not limited to, molluscum contagiosum, warts, herpes simplex virus infections such as cold sores, kanker sores and genital herpes.

In another embodiment, the powder comprising core/shell particles is used, with or without other antiparasitic active agents, to treat and prevent parasitic infections, including, but not limited to, hookworm infection, lice, scabies, sea bathers' eruption and swimmer's itch.

In one embodiment, the powder comprising core/shell particles is administered to treat ear infections (such as those caused by streptococcus oneumoniae), rhinitis and/or sinusitis (such as caused by Haemophilus influenzae, Moraxella catarrhalis, Staphylococcus aureus and Streptococcus pneumoniae), and strep throat (such as caused by Streptococcus pyogenes).

In one embodiment, the powder comprising core/shell particles is orally administered to an animal (*e.g.,* as animal feed) or a human (*e.g.,* as a dietary supplement) to prevent outbreaks of food borne illnesses (*e.g.,* stemming from food borne pathogens such as Campylobacter jejuni, Listeria monocytogenes, and Salmonella enterica).

**Topical Nail Treatment**

The powder comprising core/shell particles can also be used to stimulate nail growth, enhance nail strength, and reduce nail infection or discoloration. The powder comprising core/shell particles can be incorporated into compositions for the treatment of onychomychosis with actives such as, but not limited to miconazole, econazole, ketoconazole, sertaconazole, itraconazole, fluconazole, voricoriazole, clioquinol, bifoconazole, terconazole, butoconazole, tioconazole, oxiconazole, sulconazole, saperconazole, clotrimazole, undecylenic acid, haloprogin, butenafine, tolnaftate, nystatin, ciclopirox olamine, terbinafine, amorolfine, naftifine, elubiol, griseofulvin, and their pharmaceutically acceptable salts and prodrugs. The powder comprising core/shell particles can be incorporated into compositions for improving the look and feel of nails with ingredients such as, but not limited to: biotin, calcium panthotenate, tocopheryl acetate, panthenol, phytantriol, cholecalciferol, calcium chloride, Aloe Barbadensis (Leaf Juice), silk protein, soy protein, hydrogen peroxide, carbamide peroxide, green tea extract, acetylcysteine and cysteine.

**Topical Treatment for Hair, Hair Follicles and Scalp Skin**

The powder comprising core/shell particles can be combined with certain active agents for the growth of hair, or improving or thickening of hair of the scalp, eye brow or eye lash, may be used to treat hair conditions topically. Compositions containing drug(s) and/or active agents to stimulate hair grow and/or prevent hair loss, including, but not limited to, minoxidil, finasteride, or lumigan may be employed.

The powder comprising core/shell particles has a unique advantage over conventional hair treatment compositions due to its excellent flowability. For example, the powder can easily reach the scalp through thinned hair in the case of alopecia treatment. The powder is easily broken by gentle rubbing with a hand or comb, releasing the active agent (*e.g.,* minoxidil) to the skin near hair follicles without loss of the active onto the hair shafts, disturbing the style of the hair, or causing an undesirable hair appearance as conventional liquid gel, aerosol, foam, or spray products may do.

**Topical Compositions for Pain and Itch**

The powder comprising core/shell particles may contain certain analgesic active agents and as such may be prepared for topical treatment of pain, such as pain at or from the back, shoulder, joints, muscle sore/pain, menstrual cramps, or pain from cold sore or canker sore. Active agents to relieve pain include, but are not limited to, NonSteroidal Anti-Inflammatory Drugs (NSAIDs) such as ibuprofen, naproxen, salicylic acid, ketoprofen, and diclofenac. Other topical analgesic active agents for treating pain and itch include, but are not limited to, methyl salicylate, menthol, trolamine salicylate, capsaicin, lidocaine, benzocaine, pramoxine hydrochloride, and hydrocortisone.

**Ingestible Compositions**

Ingestible compositions, suitable for ingestion by a mammal such as a human, may be made using the powder of the invention.

In one embodiment, such an ingestible composition contains a safe and effective amount of (i) at least active agent or drug, and (ii) the powder comprising core/shell particles within which or with which the active agent or drug is located. The active agent may belong to any drug category for any treatment, including as an oral medicine, or may be a nutritional supplement. In one embodiment, the ingestible composition contains, per dosage unit (*e.g.,* powder, capsule, teaspoonful, or the like) an amount of the active agent necessary to deliver a dose effective for the needed treatment.

In one embodiment, the ingestible composition comprises a hard gelatin capsule filled with the powder of the invention, wherein one or more active agents are loaded into the liquid core, the shell, and/or outside the powder but inside the hard shell gelatin capsule. In one embodiment, the composition is in unit dosage form such as unit-packaged capsules, powders, or granules.

In another embodiment, an ingestible composition comprises two or more powders of the invention each containing an active agent loaded into the liquid core or shell of each powder. This composition is particularly suitable for active agents that are chemically incompatible.

Ingestible compositions comprising active agents contained in powders of the invention are advantageous in that: (a) some or all of the active agent may be dissolved in the liquid core of the core/shell particles, thus enabling faster gastrointestinal absorption in comparison to solid dosage forms such as tablets, dry powders, or conventional dry particle-filled hard gelatin capsules; and (b) chemically incompatible active agents may be dissolved in separate powders to avoid undesirable chemical interaction/reactions but still provide the convenience and safety of a single product (such as in a hard gelatin capsule) to the patient.

Exemplary treatments using ingestible compositions containing a powder of the invention and active agents include the following.

(a) Gastro-intestinal Disorder Treatment

In one embodiment, the ingestible compositions according to the invention are used for the treatment of gastrointestinal disorders, such as ulcers, diarrhea, and gastrointestinal pain.

Active agents for treating diarrhea include, but are not limited to: bismuths (such as Bismuth Subsalicylate), Loperamide, Simethicone, Nitazoxanide, Ciprofloxacin, and Rifaximin, salts and prodrugs (such as esters) thereof.

Active agents for treating gastric ulcers include, but are not limited to: Lansoprazole, Naproxen, Esomeprazole, Famotidine, Nizatidine, Ranitidine, and Omeprazole, and salts and prodrugs thereof.

Active agents for treating intra-abdominal infections include, but are not limited to: Moxifloxacin, Ciprofloxacin, Ceftazidime, Gentamicin, Ertapenem; Cefepime, Cefoxitin, Cilastatin, Imipenem; Ceftriaxone, Clavulanate, and Ticarcillin, and salts and prodrugs thereof.

(b) Pain or Cough Treatment with Ingestible Compositions

In one embodiment, ingestible compositions according to the invention are used for treatment of pain (such as throat pain). Oral dosage forms for this purpose can be in the form of, but not limited to, hard gelatin capsules, lozenges, or spray powder. Active agents known to treat sore throat, include, but are not limited to: Acetaminophen, Dextromethorphan, Pseudoephedrine, Chlorpheniramine, Pseudoephedrine, Guaifenesin, Doxylamine, Zinc, and Ibuprofen, and salts and prodrugs thereof.

(c) Oral Supplement Ingestible Compositions

In one embodiment, ingestible compositions according to the invention, such as hard gelatin capsules or powder dosage form, are used for oral supplement products. Active agents for such purpose include vitamins and minerals, which include, but are not limited to: Dibasic Calcium Phosphate, Magnesium Oxide, Potassium Chloride, Microcrystalline Cellulose, Ascorbic Acid (Vit. C), Ferrous Fumarate, Calcium Carbonate, dl-Alpha Tocopheryl Acetate (Vit. E), Acacia, Ascorbyl Palmitate, Beta Carotene, Biotin, BHT, Calcium Pantothenate, Calcium Stearate, Chromic Chloride, Citric Acid, Crospovidone, Cupric Oxide, Cyanocobalamin (Vit. B 12), Ergocalciferol (Vit. D), Folic Acid, Gelatin, Hypromellose, Lutein, Lycopene, Magnesium Borate, Magnesium Stearate, Manganese Sulfate, Niacinamide, niacin, Nickelous Sulfate, Phytonadione (Vit. K), Potassium Iodide, Pyridoxine Hydrochloride (Vit. B), Riboflavin (Vit. B 2), Silicon Dioxide, Sodium Aluminum Silicate, Sodium Ascorbate, Sodium Benzoate, Sodium Borate, Sodium Citrate, Sodium Metavanadate, Sodium Molybdate, Sodium Selenate, Sorbic Acid, Stannous Chloride, Sucrose, Thiamine Mononitrate (Vit. B 1), Titanium Dioxide, Tribasic Calcium Phosphate, Vitamin A Acetate (Vit. A), and Zinc Oxide., and salts and prodrugs thereof.

For ingestible compositions comprising the powder of the invention, hydrophobic fumed silica, AEROSIL 972 Pharma, from EVONIK DEGUSSA CORPORATION, is particularly suitable for use as the hydrophobic particles of the shell.

**EXAMPLES**

Example 1 - Method of Making the Core-Shell Particles

A series of powders comprising different cores were made as follows. In each case, the shell was made of AEROSIL R812S Hydrophobic Fumed Silica (commercially available from Evonik Degussa Corporation).
The powders were made using an Oster Blender (model BCBG08) with the Oster Milkshake Blade (Model 6670), and Oster Blend-N-Go Cup (Model 6026). For each powder, the ingredients of the liquid core were added to the blender at room temperature and mixed for about 1 minute. Then, AEROSIL R812S Hydrophobic Fumed Silica was added to the liquid core ingredients. The contents were blended at the highest setting for about 10-20 seconds, yielding core-shell particles.
Table 2 (single phase liquid core, not according to the invention) and table 3 (emulsion liquid core) show the ingredients of the different samples.

Example 2 - Particle Size Measurement of Core-Shell Particles (Not according to the invention)

The particle size of the core-shell particles of Sample 1 was manually measured using scanning electron microscopy (SEM). The powder was taped onto an adhesive tape across a distance of 3"-6" to ensure dispersion of the particles. Particles were then measured manually on a 27" screen with a mouse. The diameter was measured as the longest visible distance across particle. The mean and median particle diameters for Sample 1, shown in Table 1 below, were 14.77 $\mu$m and 10.92 $\mu$m, respectively. Maximum and minimum particle diameters were 51.72 $\mu$m and 3.16 $\mu$m, respectively.

Table 1. Particle Size of Sample 1 (Not according to the invention)

| Sample | Core | Shell | Particle Diameter (mean) | Particle Diameter (median) | Particle Diameter (maximum) | Particle Diameter (minimum) |
|---|---|---|---|---|---|---|
| 1 | Glycerin | 5% R812S | 14.77 um | 10.92 um | 51.72 um | 3.16 um |

Example 3 - Measurement of Surface Properties of the Core-Shell Particles

The percent polar component of overall surface tension was measured for the polar liquids of each sample made in Example 1 using the Fowkes method described herein. The overall surface tension of each sample was measured five times via the Wilhelmy plate method using a Kruss Tensiometer K100. The plate used was a standard platinum plate of 19.9 mm x 0.2 mm perimeter.
The contact angle of each sample was also measured five times on a clean piece of poly(tertafluoroethylene) PTFE using a Kruss Drop Shape Analysis System DSA10. Using the equation, the dispersive surface tension component was calculated as:

$$\sigma_L{}^D = \frac{\sigma_L{}^2 \left( \cos \theta_{PTFE} + 1 \right)^2}{72}$$

where $\theta_{PTFE}$ = the contact angle measured between PTFE and the sample liquid, and $\sigma_L$ is the surface tension of the liquid core. The polar component of overall surface tension for each liquid was then determined by difference ($\sigma_L{}^P = \sigma_L - \sigma_L{}^D$). The percent surface polarity was (%= $\sigma_L{}^P$ * 100%/ $\sigma_L$).
The results are shown in Tables 2 and 3.

Table 2. Single Phase Liquid Cores (Not according to the invention)

| Sample | Liquid Core Composition | Polar Liquid Properties | | | Stable powder comprising core/shell particles? |
|---|---|---|---|---|---|
| | | $\sigma_{L, mN/m}$ | $\phi_{PTFE}$ | Percent Surface Polarity | |
| 1 | 100 parts Glycerin | 66.40 | 97.6 | 30.56 | Yes |

(continued)

| Sample | Liquid Core Composition | Polar Liquid Properties | | | Stable powder comprising core/shell particles? |
|---|---|---|---|---|---|
| | | $\sigma_{L, mN/m}$ | $\phi_{PTFE}$ | Percent Surface Polarity | |
| 2 | 100 parts Diglycerin | 63.58 | 96.4 | 30.28 | Yes |
| 3 | 10 parts Propylene Glycol/90 parts Glycerin | 55.85 | 91.5 | 26.44 | Yes |
| 4 | 100 parts Triglycerin | 56.67 | 91.9 | 26.42 | Yes |
| 5 | 5 parts Dimethylisosorbide /95 parts Glycerin | 56.43 | 91.7 | 26.21 | Yes |
| 6 | 10 parts Polyethylene Glycol 8/90 parts Glycerin | 56.17 | 91.5 | 26.02 | Yes |
| 7 | 20 parts Polyethylene Glycol 8/80 parts Glycerin | 53.86 | 90.1 | 25.46 | Yes |
| 8 | 25 parts Polyethylene Glycol 8/75 parts Glycerin | 53.12 | 89.5 | 24.93 | No |
| 9 | 15 parts Propylene Glycol/85 parts Glycerin | 53.17 | 89.4 | 24.6 | Yes |
| 10 | 20 parts Propylene Glycol/80 parts Glycerin | 50.91 | 87.6 | 23.25 | No |
| 11 | 10 parts Dimethylisosorbide/90 parts Glycerin | 50.55 | 87.3 | 23.02 | No |
| 412 | 100 parts Polyethylene Glycol 8 | 46.73 | 84.0 | 20.82 | No |
| 13 | 5 parts Hexylene Glycol /95 parts Glycerin | 44.62 | 81.9 | 19.33 | No |
| 14 | 10 parts Hexylene Glycol/90 parts Glycerin | 39.82 | 76.5 | 15.86 | No |
| 15 | 100 parts Dimethylisosorbide | 40.36 | 76.9 | 15.65 | No |
| 16 | 100 parts Propylene Glycol | 37.15 | 72.6 | 13.74 | No |
| 17 | 100% Hexylene Glycol | 29.43 | 57.8 | 3.96 | No |

Table 3. Two-Phase Liquid Cores (samples 31 to 32 not according to the invention)

| Sample | Liquid Core Composition | Polar Liquid Properties (without the non-polar phase or surfactant) | | | Stable powder comprising core/ shell particles? |
|---|---|---|---|---|---|
| | | $\sigma_{L, mN/m}$ | $\phi_{PTFE}$ | Percent Surface Polarity | |
| | | | | | |
| 18 | 99 parts Glycerin/1.0 part Silicone Fluid/1.5 parts SP-1 | 66.40 | 97.6 | 30.56 | Yes |
| X19 | 90 parts Glycerin/10 parts Silicone Fluid/0.5 parts SP-1 | 66.40 | 97.6 | 30.56 | Yes |
| 20 | 80 parts Glycerin/20 parts Silicone Fluid/1.0 part SP-1 | 66.40 | 97.6 | 30.56 | Yes |

(continued)

| Sample | Liquid Core Composition | Polar Liquid Properties (without the non-polar phase or surfactant) | | | Stable powder comprising core/ shell particles? |
|---|---|---|---|---|---|
| | | $\sigma_{L, mN/m}$ | $\phi_{PTFE}$ | Percent Surface Polarity | |
| 21 | 70 parts Glycerin/30 parts Silicone Fluid/1.5 parts SP-1 | 66.40 | 97.6 | 30.56 | Yes |
| 22 | 55 parts Glycerin/45 part Silicone Fluid/2.25 parts SP-1 | 66.40 | 97.6 | 30.56 | Yes |
| 23 | 60 parts Glycerin/40 parts Mineral Oil/2.0 parts SP-1 | 66.40 | 97.6 | 30.56 | Yes |
| 24 | 60 parts Glycerin/40 parts Baby Oil/ 2.0 parts SP-1 | 66.40 | 97.6 | 30.56 | Yes |
| 25 | 90 parts Glycerin/10 parts Silicone Fluid/ 0.05 parts PS-111 | 66.40 | 97.6 | 30.56 | Yes |
| 26 | 90 parts Glycerin/10 parts Silicone Fluid/1.0 part PS-111 | 66.40 | 97.6 | 30.56 | Yes |
| 27 | 90 parts Glycerin/10 parts Silicone Fluid/0.5 parts TWEEN 80 | 66.40 | 97.6 | 30.56 | Yes |
| 28 | 5 parts Dimethylisosorbide /95 parts Glycerin/10 parts Silicone Fluid/1.0 part SP-1 | 56.43 | 91.7 | 26.21 | Yes |
| 29 | 10 parts Polyethylene Glycol /90 parts Glycerin/10 parts Silicone Fluid/1.0 part SP-1 | 56.17 | 91.5 | 26.02 | Yes |
| 30 | 10 parts Polyethylene Glycol /90 parts Glycerin/10 parts Silicone Fluid/0.5 parts TWEEN 80 | 56.17 | 91.5 | 26.02 | Yes |
| 31 | 10 parts Dimethylisosorbide/90 parts Glycerin/10 parts Silicone Fluid/1.0 part SP-1 | 50.55 | 87.3 | 23.02 | No |
| 32 | 10 parts Hexylene Glycol /90 parts Glycerin/ 10 parts Silicone Fluid/1.0 part SP-1 | 39.82 | 76.5 | 15.86 | No |

Notes in Table 3:
INUTEC SP-1 is inulin lauryl carbamate manufactured by Beneo.
Baby Oil is JOHNSON'S Baby Oil manufactured by Johnson and Johnson (Skillman, NJ).
PS-111 is sodium hydrolyzed potato starch dodecenylsuccinate manufactured by Akzo Nobel.
TWEEN 80 is polysorbate 80 manufactured by Spectrum.

The data shows that powders comprising core/shell particles can be made using a variety of anhydrous, polar liquids in the core. Single phase and emulsions can be employed as the liquid core. Those liquids providing a percent polar component of overall surface tension of at least about 24 % resulted in formation of core-shell particles that maintained their stabilities once formed.

Example 4 - Core-Shell Particles with Salicylic Acid (not according to the invention)

A powder according to the invention containing the active agent salicylic acid was made as follows.
186 g glycerin, 10.0 g propylene glycol, and 4.0 g salicylic acid were added to a beaker and mixed under slight heat until the salicylic acid dissolved. 196.9 g of this mixture and 10.4 g hydrophobic fumed silica (Evonik, R812S) were added to the blender and blended as described above. The contents were converted core-shell particles.

Example 5 - Core-Shell Particles formulated with Hexyl Resorcinol (not according to the invention)

A powder according to the invention containing the active agent hexyl resorcinol was made as follows.
212 g glycerin and 2.14 g hexyl resorcinol were added to a beaker and mixed until a uniform solution was obtained. 200 g of this mixture and 10.5 g hydrophobic fumed silica (Evonik, R812S) were added to a blender and blended as described above. The contents were converted core-shell particles.

**Claims**

1. A powder comprising core/shell particles having an average particle size of less than 1000 microns, each core/shell particle comprising: 1) a liquid core that is an emulsion containing 5% by weight or less of water, wherein the emulsion includes a continuous phase comprising a polar liquid having a percent surface polarity of at least 24% as determined using the method of Fowkes referenced in the description and one or more dispersed phases comprising a hydrophobic component, wherein the liquid core comprises at least one hydrophobically modified polysaccharide and 2) a shell comprising hydrophobic particles.

2. The powder of claim 1, wherein the hydrophobic particles comprise hydrophobic fumed metal oxide.

3. The powder of claim 1, wherein the hydrophobic particles comprise hydrophobic fumed silica.

4. The powder of claim 1, wherein the polar liquid comprises a polyol selected from the group consisting of glycerols, polyglycerols, glycols, polyglycols, and mixtures thereof.

5. The powder of claim 4, wherein the polyol is selected from the group consisting of glycerol, diglycerol, triglycerol, tetraglycerol, polyglycerols having more than 4 glycerol groups, and mixtures thereof.

6. The powder of claim 1, wherein the liquid core comprises 0.01% to 20% by weight of hydrophobically modified polysaccharide.

7. The powder of claim 1, wherein the polar liquid comprises at least 50 percent by weight of a glycerol, polyglycerol, or mixture thereof

8. The powder of claim 1, wherein the liquid core further comprises an active agent.

9. A personal care composition comprising a powder as defined in claim 1.

10. The composition of claim 9, further comprising a second powder.

11. The composition of claim 10, wherein the second powder comprises an ingredient selected from the group consisting of silica, fumed silica, starch powders, clays, synthetic and natural fibers, talc, and waxes.

12. The composition of claim 10, wherein the second powder comprises an active agent.

**Patentansprüche**

1. Pulver, umfassend Kern/Mantel-Partikel mit einer durchschnittlichen Partikelgröße von weniger als 1000 Mikrometern, wobei jedes Kern/Mantel-Partikel umfasst: 1) einen flüssigen Kern, der eine Emulsion ist, die 5 Gew.% oder weniger Wasser enthält, wobei die Emulsion eine kontinuierliche Phase, die eine polare Flüssigkeit mit einer prozentualen Oberflächenpolarität von mindestens 24 % umfasst, bestimmt gemäß der Methode nach Fowkes, die in der Beschreibung genannt ist, und eine oder mehrere dispergierte Phasen einschließt, die eine hydrophobe Komponente umfassen, wobei der flüssige Kern mindestens ein hydrophob modifiziertes Polysaccharid umfasst, und 2) einen Mantel, der hydrophobe Partikel umfasst.

2. Pulver nach Anspruch 1, wobei die hydrophoben Partikel hydrophobes pyrogenes Metalloxid umfassen.

**3.** Pulver nach Anspruch 1, wobei die hydrophoben Partikel hydrophobes pyrogenes Siliciumdioxid umfassen.

**4.** Pulver nach Anspruch 1, wobei die polare Flüssigkeit ein Polyol ausgewählt aus der Gruppe bestehend aus Glycerinen, Polyglycerinen, Glykolen, Polyglykolen und Mischungen davon umfasst.

**5.** Pulver nach Anspruch 4, wobei das Polyol ausgewählt ist aus der Gruppe bestehend aus Glycerin, Diglycerin, Triglycerin, Tetraglycerin, Polyglycerinen mit mehr als 4 Glyceringruppen sowie Mischungen davon.

**6.** Pulver nach Anspruch 1, wobei der flüssige Kern 0,01 Gew.% bis 20 Gew.% hydrophob modifiziertes Polysaccharid umfasst.

**7.** Pulver nach Anspruch 1, wobei die polare Flüssigkeit mindestens 50 Gewichtsprozent eines Glyerins, Polyglycerins oder einer Mischung davon umfasst.

**8.** Pulver nach Anspruch 1, wobei der flüssige Kern des Weiteren ein aktives Mittel umfasst.

**9.** Körperpflegezusammensetzung, umfassend ein Pulver wie in Anspruch 1 definiert.

**10.** Zusammensetzung nach Anspruch 9, die des Weiteren ein zweites Pulver umfasst.

**11.** Zusammensetzung nach Anspruch 10, wobei das zweite Pulver einen Bestandteil ausgewählt aus der Gruppe bestehend aus Siliciumdioxid, pyrogenem Siliciumdioxid, Stärkepulvern, Tonen, synthetischen und natürlichen Fasern, Talkum und Wachsen umfasst.

**12.** Zusammensetzung nach Anspruch 10, wobei das zweite Pulver des Weiteren ein aktives Mittel umfasst.


**Revendications**

**1.** Poudre comprenant des particules ayant une structure noyau/enveloppe dont la taille de particule moyenne est inférieure à 1 000 microns, chaque particule ayant une structure noyau/enveloppe comprenant : 1) un noyau liquide qui est une émulsion contenant 5 % en poids ou moins d'eau, l'émulsion contenant une phase continue comprenant un liquide polaire ayant une polarité de surface, exprimée en pourcentage, d'au moins 24 %, la polarité de surface exprimée en pourcentage étant déterminée en utilisant la méthode de Fowkes énoncée dans la description et une ou plusieurs phases dispersées comprenant un constituant hydrophobe, le noyau liquide comprenant au moins un polysaccharide modifié pour devenir hydrophobe et 2) une enveloppe comprenant des particules hydrophobes.

**2.** Poudre selon la revendication 1, dans laquelle les particules hydrophobes comprennent un oxyde de métal pyrogéné hydrophobe.

**3.** Poudre selon la revendication 1, dans laquelle les particules hydrophobes comprennent de la silice pyrogénée hydrophobe.

**4.** Poudre selon la revendication 1, dans laquelle le liquide polaire comprend un polyol sélectionné dans le groupe constitué de glycérols, de polyglycérols, de glycols, de polyglycols, et de mélanges de ceux-ci.

**5.** Poudre selon la revendication 4, dans laquelle le polyol est sélectionné dans le groupe constitué du glycérol, du diglycérol, du triglycérol, du tétraglycérol, de polyglycérols comportant plus de 4 groupes glycérol, et de mélanges de ceux-ci.

**6.** Poudre selon la revendication 1, dans laquelle le noyau liquide comprend 0,01 % à 20 % en poids du polysaccharide modifié pour devenir hydrophobe.

**7.** Poudre selon la revendication 1, dans laquelle le liquide polaire comprend au moins 50 % en poids d'un glycérol, d'un polyglycérol, ou d'un mélange de ceux-ci.

**8.** Poudre selon la revendication 1, dans laquelle le noyau liquide comprend en outre un principe actif.

9. Composition de soins d'hygiène personnelle comprenant une poudre telle que définie dans la revendication 1.

10. Composition selon la revendication 9, comprenant en outre une deuxième poudre.

11. Composition selon la revendication 10, dans laquelle la deuxième poudre comprend un ingrédient sélectionné dans le groupe constitué de la silice, de la silice pyrogénée, de poudres d'amidon, d'argiles, de fibres synthétiques et de fibres naturelles, du talc, et de cires.

12. Composition selon la revendication 10, dans laquelle la deuxième poudre comprend un principe actif.

FIG. 1

FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3393155 A
- US 6290941 B
- US 20120315312 A1
- WO 2011075418 A
- US 6174533 B
- US 6534647 B
- US 8258250 B
- US 7417020 B
- US 20110082105 A1
- US 20110082290 A1
- WO 2011076518 A

### Non-patent literature cited in the description

- **ESHTIAGHI et al.** *Powder Technology,* 2012, vol. 223, 65-76
- **FOWKES.** *Journal of Achievements in Materials and Manufacturing Engineering,* 2007, vol. 24 (1), 137-145
- Measurement of Interfacial Tension in Fluid-Fluid Systems. **DERELINCH.** Encyclopedia of Surface and Colloid Science. Marcel Dekker, Inc, 2002, 3152-3166
- **F.M. FOWKES.** *Journal of Physical Chemistry,* 1963, vol. 67, 2538-2541
- **O. CARRIER et al.** Inverse emulsions stabilized by a hydrophobically modified polysaccharide. *Carbohydrate Polymers,* 2011, vol. 84, 599-604
- **C. CLARO et al.** Surface tension and rheology of aqueous dispersed systems containing a new hydrophobically modified polymer and surfactants. *International Journal of Pharmaceutics,* 2008, vol. 347, 45-53
- **T. TADROS.** Polymeric Surfactants in Disperse Systems. *Advances in Colloid and Interface Science,* 2009, vol. 147 (148), 281-299
- **R.Y. LOCHHEAD ; S. JONES.** *Polymers in Cosmetics: Recent Advances,* July 2004, Happi.com
- **L. FORNY.** Influence of mixing characteristics for water encapsulation by self-assembling hydrophobic silica nanoparticles. *Powder Technology,* 2009, vol. 189, 263-269